# EUROPEAN PATENT SPECIFICATION

(11) **EP 4 008 365 B1**
(45) Date of publication and mention of the grant of the patent: **27.12.2023**
(21) Application number: 21207795.2
(22) Date of filing: 11.11.2021
(51) Int. Cl.: A61L 9/12, A45D 34/00, B65D 47/00

(54) **FRAGRANCE DIFFUSER FOR ENVIRONMENTS**
DUFTSPENDER FÜR UMGEBUNGEN
DIFFUSEUR DE PARFUM POUR ENVIRONNEMENTS

(30) Priority: 27.11.2020 IT 202000028859
(43) Date of publication of application: 08.06.2022
(73) Proprietor: Fueguia S.r.l., 20121 Milano MI (IT)
(72) Inventor: Bedel, Julian Francisco, 20121 Milano MI (IT)
(74) Representative: Perani & Partners S.p.A.

(56) References cited:
- EP-A1- 2 653 171
- WO-A1-2009/058872
- WO-A1-2011/120073
- WO-A1-2012/026310
- WO-A1-2012/029504
- GB-A- 2 526 366

## Description

### FIELD OF APPLICATION

The present invention relates to a fragrance diffuser for environments.

Specifically, the fragrance diffuser for environments of the present invention is of the type with sticks.

### Description of the Technical Background

It is known in the art to make a fragrance diffuser for environments of the type with sticks.

A known fragrance diffuser for environments comprises a container, made of transparent glass or plastic, containing a liquid fragrance for environments. The container, which is in the form of a bottle, vase or jar, has an upper mouth, which can be closed by means of a special cap to preserve the fragrance and prevent the evaporation thereof when the diffuser is not in use. The cap can be removed so that wooden sticks are inserted into the container through the mouth. The sticks are then immersed in the fragrance and, by soaking therein, release it into the air by evaporation and diffuse it into the surrounding environment.

Fragrance diffusers for environments are disclosed in documents WO 2012/029504 A1, WO 2012/026310 A1 and WO 2009/058872 A1.

### Problems of the Background Art

In the prior art, the diffuser container must be discarded once the fragrance is exhausted or alternatively it is possible to fill it with new fragrance poured by means of a special refill. In turn, the refill bottle must be disposed of. Furthermore, a further drawback is that the diffuser container cannot be used for fragrances other than the original one because, by using a different fragrance, the latter would be contaminated by residues of the previous one. To overcome this disadvantage, it is necessary to clean and wash the container very carefully to remove any possible trace of the fragrance used previously. Therefore, if the user intends to change the fragrance before the one contained in the container is finished, he must have a second container with a different fragrance inside.

Furthermore, the diffusers of the prior art, once opened, are difficult to transport as the liquid may leak from the cap.

In addition, the wooden sticks must often be replaced as they are subject to deterioration. The same sticks must be replaced when changing the fragrance to be diffused since they remain impregnated with the fragrance. Furthermore, the fragrance-impregnated wooden sticks remain damp for a long time and are therefore impractical to transport.

### SUMMARY OF THE INVENTION

The object of the invention in question is to make a fragrance diffuser for environments capable of overcoming the drawbacks of the prior art.

A further object of the invention in question is to make a kit comprising a fragrance diffuser for environments capable of overcoming the drawbacks of the prior art.

A further object of the present invention is to provide a method for assembling and using the aforesaid kit.

### Advantages of the Invention

The main advantage of the present invention is that of being able to have a diffuser in which the container is provided with a cap placed to close the lower mouth thereof, the cap having a seat shaped to accommodate and support a refill bottle. The seat allows to stably support the refill bottle of the fragrance both in use and during the replacement/refilling step of the bottle, i.e., when the cap is screwed or unscrewed.

Thanks to an embodiment, it is possible to obtain a fragrance diffuser for environments which allows greater versatility in use with respect to the known diffusers.

Thanks to an embodiment, it is possible to make a fragrance diffuser for environments which can be used with different interchangeable fragrances at any time.

Thanks to an embodiment, it is possible to make a fragrance diffuser for environments which is easy to transport.

The above advantages also apply to a kit comprising the diffuser of the present invention.

### BRIEF DESCRIPTION OF THE DRA WINGS

The features and advantages of the present invention will become apparent from the following detailed description of a possible practical embodiment thereof, illustrated by way of non-limiting example in the accompanying drawings, in which:
- figure 1a shows a fragrance diffuser for environments of the present invention in a first operating configuration,
- figure 1b shows the diffuser of figure 1a in a second operating configuration,
- figure 1c shows a component of the diffuser of figure 1a and 1b in a third operating configuration together with a component of a kit according to the present invention,
- figure 1d shows the diffuser of figure 1a-1c in a fourth operating configuration together with the component of the kit shown in figure 1c,
- figure 2 shows a kit comprising a fragrance diffuser for environments of the present invention in the use configuration,
- figure 3A shows a detail of the diffuser of figure 2,
- figure 4 shows a top view of the diffuser of figure 1a.

The diffuser and the kit illustrated in the accompanying drawings are to be understood as schematically depicted, not necessarily to scale and not necessarily with the proportions depicted between the various constituent elements.

### DETAILED DESCRIPTION

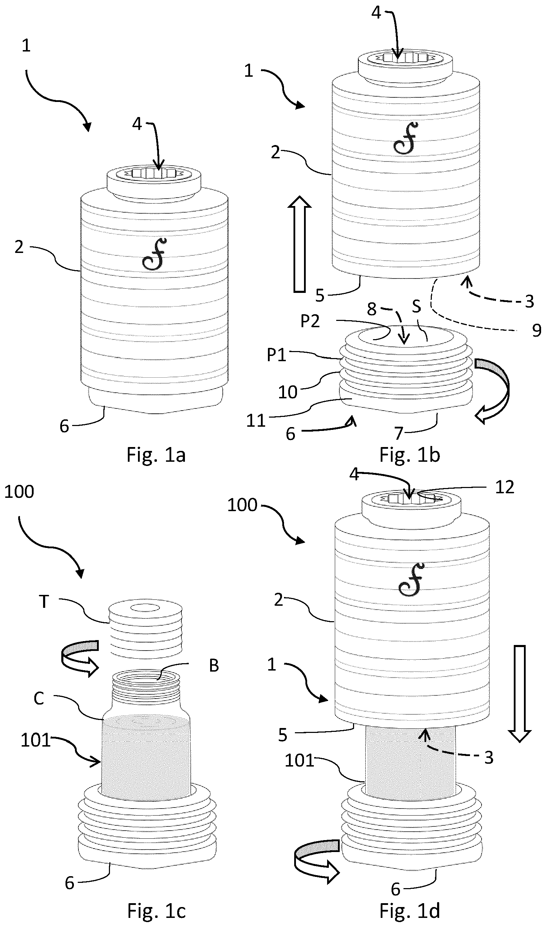

The present invention relates to a fragrance diffuser 1 for environments, illustrated in the accompanying drawings and in particular in figures 1a-1d.

The diffuser comprises a container body 2 defining an inner cavity 3 (not directly visible in the accompanying drawings, thus identified with the dashed line in figures 1b and 1d). The inner cavity 3 is shaped to accommodate a fragrance refill bottle 101 when the diffuser 1 is in use. The container body 2 is provided with an upper mouth 4. The upper mouth is shaped to allow for the insertion of a plurality of sticks 102 for diffusing fragrances into the air when the diffuser 1 is in use. The diffuser 1 comprises a lower mouth 5, opposite the upper mouth 4. The lower mouth 5 is shaped to allow the insertion of the bottle 101 to be housed in the inner cavity 3, when the diffuser 1 is in use. It should be noted here that the bottle 101 and the sticks 102 are not components of the diffuser 1 but, as it will be clearer hereinafter, they are components of a kit 100 according to the present invention.

The diffuser 1 comprises a cap 6 constrainable to the container body 2 to close the lower mouth 5. The cap 6 comprises an outer face 7 intended to come into contact with an outer support surface, and an opposite inner face 8 adapted to accommodate and support the refill bottle 101. In other words, the outer face 7 is adapted to come into contact with a flat outer support surface, and also being substantially flat it allows to keep the diffuser 1 stable on the support surface. The inner face 8 (not directly visible in the figures, thus identified with the dashed line) is shaped to accommodate and support the refill bottle 101 providing it with a stable support base, as for example shown in figures 1c and 1d.

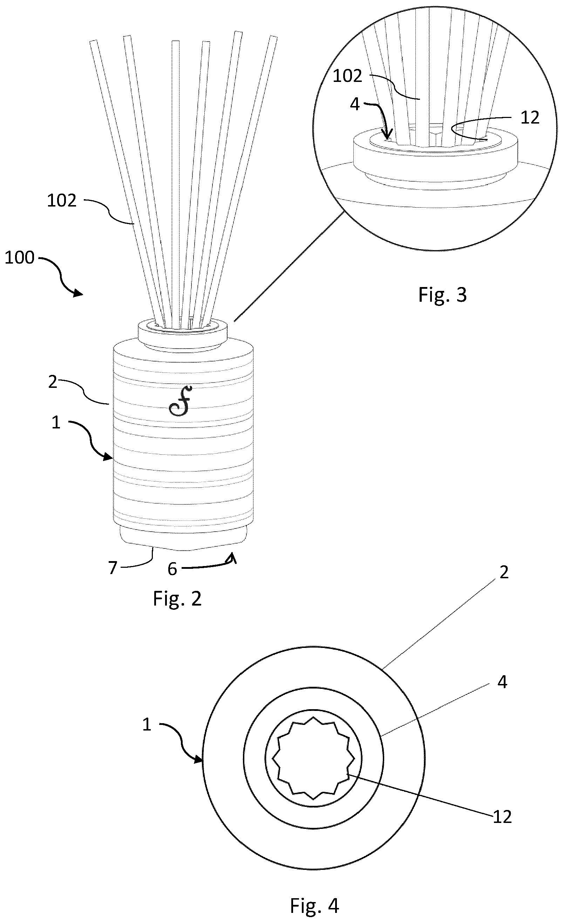

It should be noted that the diffuser 1 has two operating configurations: a first configuration in which the cap 6 is constrained to the container body 2; and a second configuration in which the cap 6 is released from the container body 2. In the first operating configuration, shown in figures 1a and 2, the diffuser 1 is closed and, when it contains a bottle 101, can be used for the diffusion of fragrances for environments. Instead, when the diffuser 1 is in the second operating configuration, shown in figures 1b-1d, it is disassembled (i.e., cap 6 released from the container body 2), and it is possible to place a bottle 101 on the cap 6 so that it can be inserted into the container body 2 once the diffuser 1 is returned to the first configuration for the diffusion of fragrances for environments.

The cap 6 is constrainable to the container body 2 to close the lower mouth 2 by threaded coupling.

The container body 2 comprises a first threaded portion 9 at the lower mouth 5 (the first threaded portion 9 is not directly visible in the accompanying drawings, thus it is identified with the dashed line in figure 1b). Furthermore, the cap 6 comprises a second threaded portion 10 intended to couple with the first threaded portion 9 to screw the cap 6 to the container body 2.

The cap 6 comprises a seat S defined between the second threaded portion 10 and the inner face 8. The seat S is shaped to accommodate and support a refill bottle 101, as shown in figures 1c and 1d. Advantageously, the seat S allows a refill bottle 101 to be permanently housed so as to allow an easy and safe replacement or refill of the same bottle 101 when the cap 6 is screwed or unscrewed. Preferably, the second threaded portion 10 defines an annular portion which together with the inner face 8 defines the seat S. Still preferably, the second threaded portion 10 has an annular shape, and has an outer wall P1 in which the thread is made which couples with the first threaded portion 9, and an opposite inner wall P2 which together with the inner face 8 defines the seat S.

Preferably, the cap 6 has an ergonomically shaped gripping portion 11 or in any case adapted to facilitate the gripping thereof by a user. More preferably, the gripping portion 11 has a hexagonal profile. The cap 6 having the hexagonal gripping portion 11 is easily grasped by a user to screw/unscrew the cap 6 from the container body 2.

According to an embodiment, the container body 2 comprises a plurality of seats 12 placed at the upper mouth 4 and defined by an alternating succession of recesses and reliefs, as shown in detail in figures 3 and 4. Each seat 12 is shaped to accommodate the profile of a respective stick 102 to keep the sticks 102 spaced apart from each other. Advantageously, when the diffuser 1 is in use the sticks remain well spaced apart so as to promote and favour the evaporation of the fragrance and the diffusion thereof in the surrounding environment. Preferably, there are twelve seats 12 and more preferably they are equidistant from each other along the lip of the upper mouth 4.

According to a preferred embodiment. The diffuser 1 is made of bronze. In detail, both the container body 2 and the cap 6 are made of bronze. Advantageously, the device 1 is very stable on a support surface during use.

According to the preferred solution of the invention shown in the accompanying drawings, the container body 2 is of substantially cylindrical shape, and both the upper mouth 4 and the lower mouth 5 are circular in shape. Preferably, the inner cavity 3 is cylindrical so as to accommodate a suitably sized bottle 101 (figures 1c and 1d).

Preferably, the diffuser 1 has dimensions such as to be pocket-sized. More preferably, the diffuser 1 as a whole has a height of 58 mm, and a diameter of 34 mm in the case of a cylindrical shape. Also preferably, the bottle 101 which can be housed in the device 1 can contain 10 ml of liquid fragrance.

Advantageously, the diffuser 1 of the present invention can be easily transported by a user and can therefore be positioned and used in any environment. In fact, given the compact size, the diffuser 1 can be used to diffuse the fragrance in a limited area, not in a large room but in a personal space. For example, the fragrance can be diffused near a desk or bedside table on which the device 1 is placed and used. This makes it possible to use the diffuser 1 in any context, as the fragrance is perceived only by the person who is in the immediate vicinity of the device 1, without however being particularly evident to other people who are in the same environment. The user can therefore choose his favourite fragrance, to be diffused for his personal, private and intimate space. Furthermore, the same diffuser 1 can be used with different fragrances simply by replacing the bottle 101 insertable therein. In other words, it is possible to unscrew the cap 6 from the container body 2 to replace a bottle 101 containing a first fragrance with a second bottle 101 containing a second fragrance (fig. 1a-1d).

The present invention also relates to a kit 100 for the diffusion of fragrances for environments.

The kit 10 comprises a diffuser 1 of fragrances for environments according to the present invention.

Furthermore, the kit comprises at least one refill bottle 101 adapted to contain a fragrance in liquid format. Preferably, the bottle 101 contains a fragrance in liquid format therein. The bottle 101 is configured to be accommodated and housed inside the diffuser 1 to diffuse the fragrance into the air. Still preferably, the refill bottle 101 comprises a body C having a mouth B and a removable cap T placed at the closure of the mouth B. The bottle 101 is adapted to be accommodated and housed inside the inner cavity 3 of the container body 2 of the diffuser 1, while it is supported and maintained inside the container body 2 thanks to the cap 6 when the latter is constrained to the container body 2.

According to a preferred solution of the invention shown in figure 2, the kit 100 comprises a plurality of sticks 102 partially insertable inside the bottle 101 through the upper mouth 4 thereof when housed in the container body 2 of the diffuser 1. Preferably, the plurality of sticks 102 comprises six sticks. Also preferably, each stick 102 has a length of 140 mm and a diameter of 1.7 mm.

According to a preferred embodiment of the kit 100, the diffuser 1 is made of bronze. Still preferably, the sticks 102 are made of porcelain. Advantageously, porcelain allows, thanks to the intrinsic porosity thereof, to favour the diffusion of the fragrance. Furthermore, the porcelain sticks 102 are reusable as they are washable and do not impregnate with the fragrance, and also are not subject to deformation and deterioration as instead occurs with the wooden sticks of the known art.

Advantageously, the kit 100 of the present invention can be easily transported. Furthermore, the same diffuser 1 can be used with different fragrances simply by replacing the bottle 101 inserted therein.

The present invention also relates to a method for assembling and using a kit 100 according to the present invention.

The method comprises the step of releasing the cap 6 from the container body 2, to bring the diffuser 1 from the first to the second operating configuration. According to a preferred embodiment, the step of releasing the cap 6 from the container body 2 is performed by unscrewing the cap 6 from the container body 2.

The method comprises the step of placing the refill bottle 101 on the inner face 8 of the cap 6. According to a preferred embodiment, the step of placing the refill bottle 101 on the inner face 8 of the cap 6 includes inserting the bottle 101 into the seat S of the cap 6.

The method comprises the step of constraining the container body 2 to the cap 6, bringing the diffuser from the second to the first operating configuration, to insert the refill bottle 101 into the inner cavity 3. According to a preferred solution, the step of constraining the container body 2 to the cap 6 includes screwing the container body 2 to the cap 6 or vice versa.

Preferably, the method comprises the further step of inserting the sticks 102 at least partially through the upper mouth 4 of the container body 2, and then through the mouth B of the bottle 101, so that the same can be immersed at least in part in the liquid fragrance contained in the bottle 101 housed in the inner cavity 3 of the container body 2 of the diffuser 1.

According to a preferred embodiment, the method also includes the step of arranging the sticks 102 in an orderly and spaced manner using the appropriate seats 12 obtained at the upper mouth 4 of the container body 2 of the diffuser 1.

Advantageously, the method of the present invention makes it possible to use the same kit 100, and therefore the same diffuser 1, for the alternating diffusion of different fragrances, replacing the bottle 101 with another bottle 101 containing a different liquid fragrance.

## Claims

1. Fragrance diffuser (1) for environments, comprising:
- a container body (2) defining an inner cavity (3) and provided with an upper mouth (4) through which a plurality of sticks (102) for the diffusion of fragrances in the air is insertable;
- the container body (2) comprising a lower mouth (5), opposite the upper mouth (4), through which a bottle (101) to be housed in the inner cavity (3) is insertable;
- a cap (6) constrainable to the container body (2) to close the lower mouth (5) ) by threaded coupling, the cap (6) comprising an outer face (7) intended to come into contact with an outer support surface, and an opposite inner face (8) adapted to accommodate and support the refill bottle (101) to be housed in the inner cavity (3), wherein
- the container body (2) comprises a first threaded portion (9) at the lower mouth (5);
- the cap (6) comprises a second threaded portion (10) intended to couple with the first threaded portion (9) to screw and unscrew the cap (6) with respect to the container 20 body (2),
**characterized in that**
- the cap (6) comprises a seat (S) defined between the second threaded portion (10) and the inner face (8), the seat (S) being shaped to accommodate and support the refill bottle (101).

2. Diffuser (1) according to claim 1, wherein
- the container body (2) comprises a plurality of seats (12) located at the upper mouth (4) and defined by an alternating succession of recesses and reliefs, each seat (12) being shaped to accommodate the profile of a respective stick (102) to keep the sticks (102) spaced apart from each other.

3. Diffuser (1) according to any one of the preceding claims, wherein
- the cap (6) comprises a gripping portion (11) having a hexagonal profile.

4. Kit (100) for the diffusion of fragrances for environments, comprising:
- a fragrance diffuser (1) for environments according to any one of claims 1 to 3;
- at least one refill bottle (101) containing a fragrance in liquid format, the bottle (101) being configured to be accommodated and housed inside the diffuser (1) to diffuse the fragrance into the air.

5. Kit (100) according to claim 4, comprising:
- a plurality of sticks (102) partially insertable inside the bottle (101) when housed in the diffuser (1).

6. Kit (100) according to claim 5, wherein:
- the diffuser (1) is made of bronze;
- the sticks (102) are made of porcelain.

7. Method for assembling and using a kit (100) according to one or more of claims 4 to 6, comprising the steps of:
- releasing the cap (6) from the container body (2),
- placing the refill bottle (101) on the inner face (8) of the cap (6);
- constraining the container body (2) to the cap (6) to insert the refill bottle (101) in the inner cavity (3).

## Patentansprüche

1. Duftspender (1) für Umgebungen, umfassend:
- einen Behälterkörper (2), der einen inneren Hohlraum (3) definiert und mit einer oberen Mündung (4) versehen ist, durch die eine Vielzahl von Stäben (102) zur Diffusion von Duftstoffen in die Luft eingeführt werden kann;
- den Behälterkörper (2), der eine untere Mündung (5) gegenüber der oberen Mündung (4) umfasst, durch die eine im inneren Hohlraum (3) unterzubringende Flasche (101) einführbar ist;
- eine Kappe (6), die mit dem Behälterkörper (2) festgespannt werden kann, um die untere Mündung (5) durch Gewindekupplung zu verschließen, wobei die Kappe (6) eine Außenfläche (7), die dazu bestimmt ist, mit einer äußeren Stützfläche in Kontakt zu kommen, und eine gegenüberliegende Innenfläche (8) aufweist, die dazu geeignet ist, die im inneren Hohlraum (3) unterzubringende Nachfüllflasche (101) aufzunehmen und zu stützen, wobei
- der Behälterkörper (2) einen ersten Gewindeabschnitt (9) an der unteren Mündung (5) umfasst;
- die Kappe (6) einen zweiten Gewindeabschnitt (10) umfasst, der dazu bestimmt ist, mit dem ersten Gewindeabschnitt (9) zu koppeln, um die Kappe (6) in Bezug auf den Behälterkörper (2) zu schrauben und abzuschrauben, **dadurch gekennzeichnet, dass**
- die Kappe (6) einen Sitz (S) umfasst, der zwischen dem zweiten Gewindeabschnitt (10) und der Innenfläche (8) definiert ist, wobei der Sitz (S) so geformt ist, dass er die Nachfüllflasche (101) aufnimmt und stützt.

2. Spender (1) nach Anspruch 1, wobei
- der Behälterkörper (2) eine Vielzahl von Sitzen (12) umfasst, die sich an der oberen Mündung (4) befinden und durch eine alternierende Abfolge von Aussparungen und Reliefs definiert sind, wobei jeder Sitz (12) so geformt ist, dass er das Profil eines jeweiligen Stabs (102) aufnimmt, um die Stäbe (102) voneinander beabstandet zu halten.

3. Spender (1) nach einem der vorhergehenden Ansprüche, wobei
- die Kappe (6) einen Griffabschnitt (11) mit einem sechseckigen Profil umfasst.

4. Kit (100) zur Diffusion von Düften für Umgebungen, umfassend:
- einen Duftspender (1) für Umgebungen nach einem der Ansprüche 1 bis 3;
- mindestens eine Nachfüllflasche (101), die einen Duftstoff in flüssigem Format enthält, wobei die Flasche (101) konfiguriert ist, um innerhalb des Spenders (1) aufgenommen und untergebracht zu werden, um den Duftstoff in die Luft zu diffundieren.

5. Kit (100) nach Anspruch 4, umfassend:
- eine Vielzahl von Stäben (102), die teilweise innerhalb der Flasche (101) eingeführt werden können, wenn sie im Spender (1) untergebracht sind.

6. Kit (100) nach Anspruch 5, wobei:
- der Spender (1) aus Bronze besteht;
- die Stäbe (102) aus Porzellan bestehen.

7. Verfahren zum Zusammenbauen und Verwenden eines Kits (100) nach einem oder mehreren der Ansprüche 4 bis 6, umfassend die folgenden Schritte:
- Lösen der Kappe (6) vom Behälterkörper (2),
- Platzieren der Nachfüllflasche (101) auf der Innenfläche (8) der Kappe (6);
- Festspannen des Behälterkörpers (2) an der Kappe (6), um die Nachfüllflasche (101) in den inneren Hohlraum (3) einzuführen.

## Revendications

1. Diffuseur de parfum (1) pour environnements, comprenant :
- un corps de récipient (2) définissant une cavité intérieure (3) et pourvu d'une ouverture supérieure (4) à travers laquelle une pluralité de bâtonnets (102) pour la diffusion de parfums dans l'air peut être insérée ;
- le corps du récipient (2) comprenant une bouche inférieure (5), opposée à l'ouverture supérieure (4), par laquelle un flacon (101) devant être logé dans la cavité intérieure (3) peut être inséré ;
- un bouchon (6) qui peut être contraint au corps du récipient (2) pour fermer l'ouverture inférieure (5) par un raccord fileté, le bouchon (6) comprenant une face extérieure (7) destinée à entrer en contact avec une surface de support extérieure, et une face intérieure (8) opposée apte à accueillir et soutenir le flacon de recharge (101) devant être logé dans la cavité intérieure (3), dans lequel
- le corps de récipient (2) comprend une première portion filetée (9) au niveau de l'ouverture inférieure (5) ;
- le bouchon (6) comprend une deuxième portion filetée (10) destinée à se coupler avec la première portion filetée (9) pour visser et dévisser le bouchon (6) par rapport au corps de récipient 20 (2),
**caractérisé en ce que**
- le bouchon (6) comprend un siège (S) défini entre la deuxième portion filetée (10) et la face intérieure (8), le siège (S) étant formé pour accueillir et soutenir le flacon de recharge (101).

2. Diffuseur (1) selon la revendication 1, dans lequel
- le corps de récipient (2) comprend une pluralité de sièges (12) situés au niveau de l'ouverture supérieure (4) et définis par une succession alternée d'évidements et de reliefs, chaque siège (12) étant formé pour accueillir le profil d'un bâtonnet (102) respectif afin de maintenir les bâtonnets (102) espacés les uns des autres.

3. Diffuseur (1) selon l'une quelconque des revendications précédentes dans lequel
- le bouchon (6) comprend une portion de préhension (11) ayant un profil hexagonal.

4. Kit (100) pour la diffusion de parfums pour environnements, comprenant :
- un diffuseur de parfum (1) pour environnements selon l'une des revendications 1 à3 ;
- au moins un flacon de recharge (101) contenant un parfum sous forme liquide, le flacon (101) étant configuré pour être logé à l'intérieur du diffuseur (1) afin de diffuser le parfum dans l'air.

5. Kit (100) selon la revendication 4, comprenant :
- une pluralité de bâtonnets (102) partiellement insérables à l'intérieur du flacon (101) lorsqu'il est logé dans le diffuseur (1).

6. Kit (100) selon la revendication 5, dans lequel :
- le diffuseur (1) est en bronze ;
- les bâtonnets (102) sont en porcelaine.

7. Procédé d'assemblage et d'utilisation d'un kit (100) selon une ou plusieurs des revendications 4 à 6, comprenant les étapes de :
- libérer le bouchon (6) du corps de récipient (2),
- placer le flacon de recharge (101) sur la face intérieure (8) du bouchon (6) ;
- contraindre le corps de récipient (2) au bouchon (6) pour insérer le flacon de recharge (101) dans la cavité intérieure (3).
